# DEMANDE DE BREVET EUROPEEN

(11) **EP 4 838 207 A1**
(43) Date de publication de la demande: **11.11.2026**
(21) Numéro de dépôt: 25174492.6
(22) Date de dépôt: 06.05.2025
(51) Int. Cl.: G01N 21/84, G01N 33/493, G01N 21/78, G01N 21/77

(54) **ANALYSEUR ET PROCÉDÉ D'ANALYSE DE PLUSIEURS SUBSTANCES PRÉDÉTERMINÉES**

(71) Demandeur: Altiscreen Sàrl, 2822 Courroux (CH)
(72) Inventeur: Miron, Simona-Melania, 68200 Mulhouse (FR); Dumea, Leonard, 68440 Steinbrunn-le-Bas (FR)
(74) Mandataire: P&TS SA (AG, Ltd.)

(57) **Abrégé**

La présente invention se rapporte à un analyseur (1) de plusieurs substances prédéterminées comprenant au moins un support de tests, au moins un dispositif optique (4) permettant de lire plusieurs bandelettes simultanément et une unité (8) de traitement des signaux. L'invention se rapporte en outre à un système comprenant un tel analyseur et un ou plusieurs accessoires et un kit d'analyse. L'invention se rapport de plus à un procédé de détection de plusieurs substances au moyen d'un tel dispositif.

## Description

### Domaine technique

La présente invention concerne un dispositif de détection et/ou de mesure de produits absorbés par un individu. En particulier, la présente invention concerne un dispositif de détection et/ou de mesure de produits illicites, tels que des drogues, potentiellement absorbés par un individu. Le dispositif objet de la présente invention permet en outre de mesurer une quantité ou une concentration d'un ou plusieurs produits absorbés par un individu. Le dispositif de la présente invention permet en outre d'identifier un ou plusieurs produits présents dans un échantillon. Le dispositif de la présente invention, du fait de ses capacités d'identification, de détection et de mesure est désigné comme un analyseur. Le dispositif objet de la présente invention permet en outre de collecter des échantillons d'analyse pour la détection et la mesure d'éventuels produits illicites qu'ils contient. L'invention concerne en outre une méthode de détection et de mesure de tels produits.

### Etat de la technique

La consommation de produits illicites représente un problème sociétal. Des risques d'accidents, d'agressivité ou d'agressions sont liés à la consommation de drogues. Des conséquences en termes de baisse de productivité ou d'absentéisme sont également identifiées. La santé des consommateurs de tels produits est également mise en danger, que ce soit sur des aspects physiologiques ou d'ordre phycologique. De nombreux développements techniques visent à identifier d'éventuels contrevenants, pour des raisons médicales et/ou d'ordre légal. En l'occurrence, les contrevenants s'exposent à des sanctions lorsqu'ils sont testés positivement par les forces de l'ordre. Cependant, les tests pratiqués à cette fin nécessitent souvent une analyse en laboratoire avant d'obtenir un résultat fiable. Ces procédés, qui doivent être certifiés pour être valables, requièrent ainsi de conduire les personnes au laboratoire pour y effectuer un ou plusieurs tests, au besoin sanguins, ce qui rend les tests compliqués à mettre en œuvre et par conséquent limite les opérations de contrôle.

Des dispositifs portables sont connus pour effectuer des tests sur le terrain, par exemple par analyse de salive. Ces dispositifs restent cependant moyennement fiables, et peuvent conduire à des erreurs de mesures si le dispositif n'est pas correctement utilisé, ou à des erreurs de lecture du résultat qui peut souvent paraître ambigu. Des téléphones portables peuvent par exemple être utilisés à cette fin, comme indiqué dans EP4010699. Les tests ainsi pratiqués sont alors souvent soumis à interprétation et ne peuvent être directement utilisés comme moyen de preuve.

En outre, les dispositifs actuellement utilisés se limitent à l'analyse d'un produit, ce qui restreint l'efficacité de détection des consommateurs de drogue. Les dispositifs permettant la détection de plusieurs produits nécessitent encore d'être calibrés pour chacun des produits testés, de manière séquentielle, ce qui complique les opérations de contrôle.

Le document EP3980780 décrit un dispositif de lecture de bandes tests au moyen d'une source de lumière contrôlée, avec plusieurs longueurs d'ondes, sur une séquence de plusieurs images. L'intensité du signal et les couleurs, perçues par un capteur CMOS ou CCD, peuvent être modulées électroniquement. Une bande de test est insérée vierge dans le dispositif puis imbibée de l'échantillon liquide. Les variations de couleur de la bande de test sont ainsi suivies avec la progression du liquide.

Il y a néanmoins matière à améliorer les dispositifs et les méthodes actuellement utilisés, en l'occurrence en termes de fiabilité et de reproductibilité, en termes de flexibilité et/ou en termes de productivité.

### Bref résumé de l'invention

Un but de la présente invention est de proposer un dispositif de détection et/ou de mesure, soit un analyseur, plus fiable que ceux actuellement utilisés. En particulier, la présente invention vise à proposer un dispositif permettant de lire un résultat de test de manière reproductible et non soumise à interprétation. Un tel dispositif doit pouvoir être utilisé sur le terrain. De préférence, un tel dispositif vise à supprimer ou au moins limiter les tests de contrôle effectués en laboratoire.

Un objectif de la présente invention est de pouvoir mettre à disposition d'agents non spécifiquement formés pour effectuer des tests biologiques un dispositif de détection et/ou de mesure, soit un analyseur, facile d'utilisation et permettant de procéder à des contrôles fiables sur le terrain.

Un objectif de la présente invention est de proposer un dispositif de détection et/ou de mesure, soit un analyseur, pouvant être légalement certifié comme fiable, de sorte que les résultats obtenus puissent être utilisés comme moyens de preuve.

Un autre but de la présente invention est de proposer un dispositif de détection et/ou de mesure, soit un analyseur, plus flexible que ceux actuellement connus. En particulier, la présente invention vise à permettre la détection de plusieurs produits de manière fiable. Elle vise en outre à améliorer les vitesses de détection des produits recherchés.

Un autre but de la présente invention est de proposer un dispositif de détection et/ou de mesure, soit un analyseur, adaptable à la détection de nouveaux produits de façon facile et rapide. En outre, il s'agit de proposer un dispositif de détection facilement adaptable aux changement de législation, notamment concernant d'éventuels seuils autorisés.

Un autre but de la présente invention est de proposer un dispositif de détection et/ou de mesure, soit un analyseur, permettant de déterminer, outre la présence ou l'absence d'un produit illicite, sa concentration ou sa quantité.

Un autre but de l'invention est de proposer une méthode de détection de produits illicite, fiable et flexible, pouvant être mise en œuvre sur le terrain.

Selon l'invention, ces buts sont atteints notamment au moyen du dispositif et de la méthode de détection objet des revendications indépendantes et décrits plus en détail dans les revendications qui en dépendent.

Cette solution présente notamment l'avantage par rapport à l'art antérieur de permettre des détections fiables, voir légalement valables, d'au moins un produit illicite, de préférence de plusieurs produits illicites, ainsi que leur identification.

### Brève description des figures

Des exemples de mise en œuvre de l'invention sont indiqués dans la description illustrée par les figures suivantes :
- Figure 1 : Représentation schématique d'un dispositif de détection et/ou de mesure selon un mode de réalisation de la présente invention,
- Figure 2 : Représentation tridimensionnelle en perspective d'un dispositif de détection et/ou de mesure selon un mode de réalisation de la présente invention,
- Figure 3 : Représentation schématique en coupe transversale d'un dispositif de détection avec tests, selon un mode de réalisation de la présente invention,
- Figure 4 : représentation d'un test selon un mode de réalisation de la présente invention,
- Figure 5 : représentation d'un accessoire selon un mode de réalisation de la présente invention,
- Figure 6 : représentation schématique d'un détail de réalisation de la présente invention

### Exemple(s) de mode de réalisation de l'invention

L'analyseur **1** selon la présente invention est adapté à la fois pour la détection et la mesure d'un ou plusieurs produits. La détection se réfère à l'identification d'un ou plusieurs produits dans un échantillon. La mesure se réfère à la détermination d'une quantité ou d'une concentration des produits détectés. Bien que la détection d'un ou plusieurs produits puisse être effectuée visuellement au moyen de tests colorés par exemple, de tels tests ne permettent pas de quantifier les produits détectés. La mesure au moyen du dispositif de mesure selon la présente invention est alors requise. L'analyseur **1** selon la présente invention comporte à cet effet des éléments de mesure. Les éléments de mesure permettent à la fois de détecter la présence d'un ou plusieurs produits et de déterminer leur quantité ou leur concentration. Les éléments de mesure comprennent en l'occurrence le boitier **10,** éventuellement pourvu d'une ouverture **120** pour y insérer un test, le support **12** permettant de maintenir le test, l'écran **11** et le ou les connecteurs **13** décrits plus en détails ci-dessous. Il comporte en outre une ou plusieurs unités de traitement **8** et des capteurs optiques **4** permettant de lire les tests, comme détaillé plus loin. Les éléments de mesures sont adaptés également à la détection automatisée des produits présents dans les échantillons analysé et peuvent ainsi confirmer ou infirmer des évaluations visuelles préalables.

L'analyseur selon la présente invention comporte en outre des éléments de détection non adaptés à la mesure des échantillons, mais permettant de collecter un ou plusieurs fluides à analyser et d'effectuer une évaluation préliminaire non automatisée. L'objectif de la présente invention est néanmoins de ne pas limiter l'analyse à une simple détection visuelle mais d'effectuer une analyse quantitative, de préférence automatisée, au moyen du dispositif ici-décrit. Les éléments de détection comprennent par exemple les bandelettes **3,** le test **2** et la capsule **34,** décrits plus en détails plus bas. L'analyseur permet d'effectuer les mesures sur la base des éléments de détection.. L'analyseur est nécessaire pour identifier la ou les substances détectée et en déterminer leur concentration. Cela permet dans ce cas d'éviter toute erreur de lecture et d'inciter à une mesure automatisée.

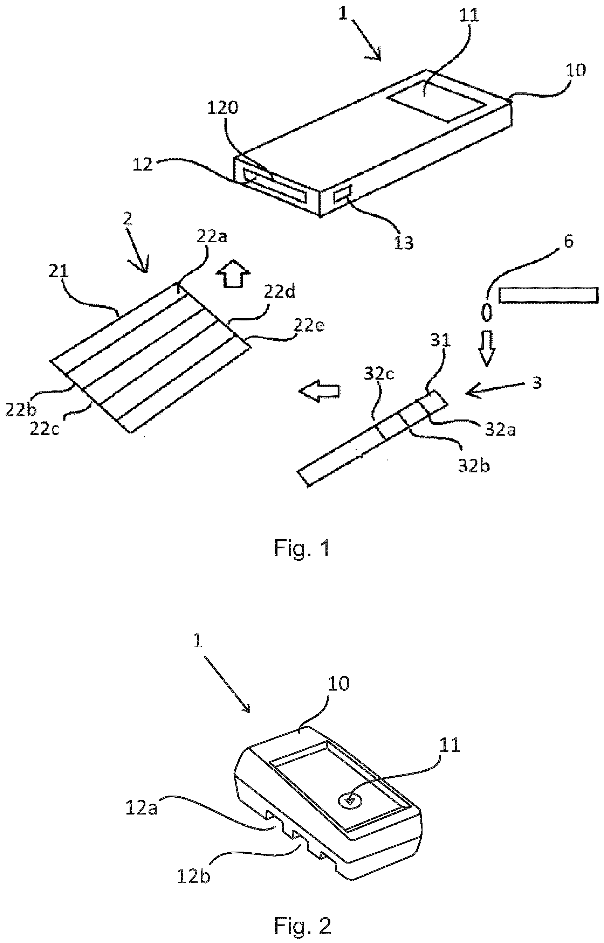

En référence à la figure 1, l'analyseur **1** selon la présente invention comporte un boitier **10** pourvu d'une ouverture **120** et d'au moins un logement **12** dans lequel un test **2** peut être inséré. Le test **2** comprend une ou plusieurs bandelettes **3** comportant une surface de lecture, avec une zone de test **31** pourvue d'un ou plusieurs réactifs adéquats pour l'identification d'un produit biologique et/ou de ses métabolites, constituant d'un échantillon d'analyse **6.** Les réactifs sont regroupés sur la bandelette **3** en plusieurs segments d'analyse **32a, 32b, 32c** dont la couleur peut varier au contact de l'échantillon d'analyse **6.** L'échantillon d'analyse **6** est de préférence une solution liquide comprenant un prélèvement de fluide corporel tel que de la salive ou de l'urine, ou directement un tel fluide corporel. En fonction de la présence ou de l'absence d'un produit recherché, un ou plusieurs des segments d'analyse **32a, 32b, 32c** peuvent changer de couleur. Un ou plusieurs des segments d'analyse **32a, 32b, 32c** peuvent servir de témoin, indiquant le bon déroulement de l'analyse. Un tel segment peut alors changer de couleur si les conditions d'analyse sont correctes, et ne pas changer de couleur si elles sont invalides. Les changements de couleur des autres segments d'analyse sont alors interprétés en conséquence.

Les segments d'analyse **32a, 32b, 32c** peuvent être sous forme de lignes transversales à la bandelette **3** ou de surfaces dédiées telles que des carrés ou des disques ou prendre toute autre apparence propre à faciliter la détection.

En fonction des produits à analyser et des bandelettes **3** utilisées le nombre de segments d'analyse peut varier. L'intensité des variations de couleur peut en outre varier en fonction des réactifs utilisés ou de la concentration du produit dans l'échantillon **6.** Bien que les changements d'aspect peuvent être identifiés à l'œil nu, les faibles variations de couleurs peuvent rendre le diagnostique imprécis. Le diagnostique peut en outre dépendre des segments d'analyse **32a, 32b, 32c** ayant réagit, ce qui complique le diagnostique humain, effectué généralement par des utilisateurs non spécialisés. Les utilisateurs peuvent en effet être des agents de police, du personnel de sécurité, un employeur ou un de ses représentants ou toute autre personne en charge de faire un test rapide sur place.

L'analyseur **1** selon la présente description est alors adapté à déterminer la présence ou l'absence d'une ou plusieurs substances à détecter. La présence ou l'absence d'une ou plusieurs substances à détecter peut être déterminée au moyen d'un seuil de détection, en particulier dans le cas où la concentration des produits détectés peut être déterminée, par exemple grâce à l'intensité des couleurs des segments d'analyse **32a, 32b, 32c,** ou grâce au nombre ou au type de segments d'analyse ayant réagit. Alternativement ou en plus, l'analyseur **1** est adapté à déterminer la concentration d'une ou plusieurs substances à détecter. La concentration peut être alors exprimée en une certaine valeur par litre de sang ou par ml de fluide collecté, par exemple une valeur comprise entre 0,1 ng/mL et 10 ng/mL ou entre 0,5 ng/mL et 5 ng/mL de fluide collecté dans l'échantillon de mesure. L'analyseur peut produire en l'occurrence les valeurs en ng de substance par ml de liquide biologique. Les fourchettes de valeurs peuvent être adaptées en fonction des substances recherchées. De préférence, l'analyseur **1** selon la présente invention permet d'identifier la présence ou l'absence, et/ou la concentration de plusieurs substances en une seule mesure. En l'occurrence, plusieurs bandelettes **3,** chacune dédiée à la détection d'une substance particulière, peuvent être lues simultanément par l'analyseur **1.** Un nombre de 2 à 10 bandelettes peuvent être lues simultanément, typiquement 7, 8 ou 9 bandelettes.

Les tests sont de préférence dédiés à la détection de substances nocives ou illicites, telles que des drogues ou des médicaments. Des substances telles que les amphétamines, les métamphétamines, les opiacés naturels ou synthétiques, le phencyclidine (PCP), la cocaïne, la marijuana, les barbituriques, le cannabis, l'ectasie, l'héroïne peuvent faire l'objet d'une détection. De préférence, les substances officiellement listées comme illicites ou répréhensibles sont ciblées. Ces substances peuvent varier d'un état à un autre. Le présent analyseur 1 permet de s'adapter facilement à la détection d'un ensemble de substances en fonction des besoins ou des règlementations.

L'analyse des bandelettes **3** est effectuée au moyen d'un ou plusieurs dispositif optique **4** aptes à identifier un ou plusieurs paramètres parmi le nombre des segments d'analyse **32a, 32b, 32c,** leur position sur la bandelette **3,** leur couleur ou leur variation de couleur, l'intensité des couleurs détectées. Un dispositif optique **4** selon la présente invention comprend au moins un capteur digital tel qu'un capteur de type CMOS ou CCD ou équivalent. Le ou les dispositifs optiques **4** sont disposés à l'intérieur du boitier **10** de sorte à rester isolés de la lumière ambiante. Typiquement le ou les dispositifs optiques 4 sont disposés dans le logement **12** du boitier.

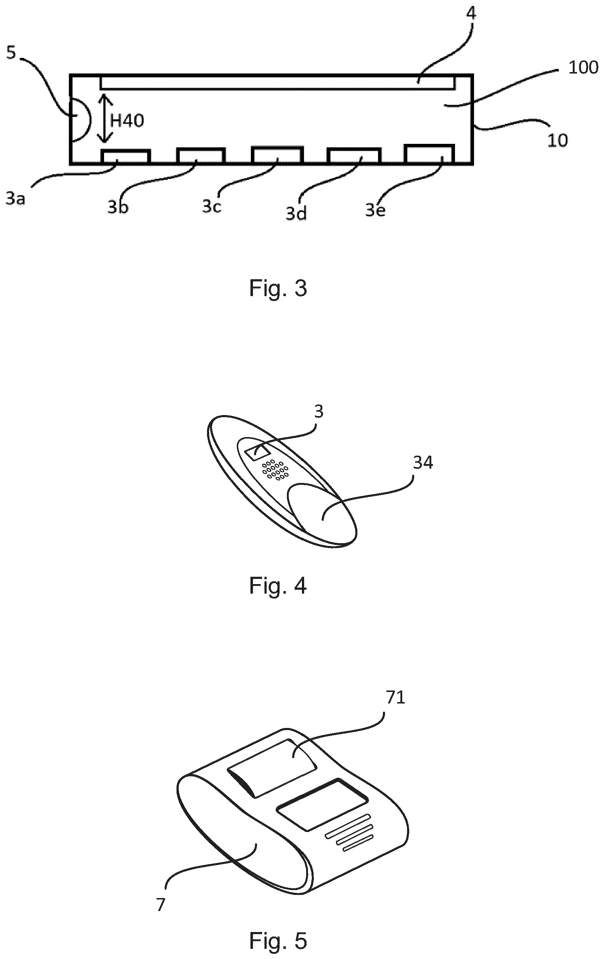

En référence à la figure 3, le logement **12** comporte une ouverture **120** dans laquelle les tests **2** peuvent être insérés dans le boitier pour y être lus. La ou les bandelettes **3** peuvent ainsi être disposées de sorte à présenter la zone de test **31,** comprenant les segments d'analyse **32a, 32b, 32c,** en vis-à-vis du ou des dispositifs optiques **4.** Selon un mode de réalisation, un dispositif optique **4** est disposé en vis-à-vis des zones de test **31** de chacune des bandelettes **3.** Alternativement, un dispositif optique unique **4** est disposé de sorte à pouvoir lire toutes les zones de test **31** des bandelettes **3** insérées dans le boitier **10.** Des solutions intermédiaires où plusieurs dispositifs optiques **4** sont disposés de sorte à lire chacun plusieurs zones de test **31** de bandelettes différentes, peut être envisagée. Dans ce dernier cas, une zone de test **31** peut être lue simultanément par deux dispositifs optiques **4.** Cette disposition peut améliorer la qualité de lecture des zones de tests **31,** notamment en moyennant le signal de plusieurs capteurs.

Selon un mode de réalisation, le logement **12** peut être obturé par un couvercle (non représenté). De la sorte, la lecture des bandelettes **3** n'est pas parasitée par la lumière ambiante. Le couvercle peut être par exemple monté sur charnière de sorte à pivoter pour laisser l'ouverture **120** libre, ou bien de type coulissant, ou encore clipsable sur le boitier. D'autres dispositions peuvent être envisagées.

Selon un mode de réalisation, l'ouverture **120** est disposée latéralement au boitier **10** de l'analyseur **1.** Ainsi, les tests **2** peuvent être insérés dans le logement **12** par cette ouverture. Alternativement, une paroi du logement **12** est amovible ou pivotable de sorte à ouvrir le logement et à disposer les tests **2** dans le logement **12.** La paroi peut ensuite être remise en place de sorte à refermer le logement **12** et permettre la lecture des tests **2.** Alternativement, un mécanisme à tiroir peut être envisagé pour ouvrir le logement **12,** une portion translatable faisant office de tiroir étant utilisée pour disposer les tests **2** et les insérer dans le logement **12** via la fermeture du tiroir.

Selon un mode de réalisation, plusieurs logement **12a, 12b** sont prévus (figure 2). Un ou plusieurs des logements de l'analyseur **1** peuvent prendre la forme d'évidements dans le boitier **10** ayant une frome tridimensionnelle adaptée pour accueillir un ou plusieurs tests **2.**

L'analyseur **1** peut comporter une ou plusieurs sources lumineuse 5. De telles sources lumineuses **5** peuvent être disposées dans le logement **12** de sorte à éclairer la zone de test **31** des bandelettes **3** pour permettre leur détection par les dispositifs optiques **4.** Dans le cas de plusieurs sources lumineuses **5** elles peuvent émettre des longueurs d'ondes identiques ou bien différentes. Les longueurs d'ondes peuvent se trouver dans le visible. Alternativement ou en plus, des longueurs d'ondes dans le domaine UV peuvent être prévues pour une lecture plus précise et/ou plus spécifique des segments d'analyse **32a, 32b, 32c.** Une source lumineuse selon la présente invention peut par exemple être de type LED ou tout équivalent. La ou les sources lumineuses **5** sont disposées de sorte à éclairer de manière homogène les zones de test **31.** Elles peuvent être par exemple disposées dans le logement **12** de façon intercalée avec les dispositifs optiques **4** lorsqu'ils sont plusieurs. Alternativement, dans le cas d'un seul dispositif optique **4,** une ou plusieurs sources lumineuse **5** peut jouxter le dispositif optique **4** de sorte à projeter la lumière perpendiculairement à la surface des bandelettes **3** lues par le ou les dispositifs optiques **4.** Alternativement ou en plus, la ou les sources lumineuses **5** peuvent être disposées de sorte à projeter une lumière incidente non perpendiculaire à la surface des bandelettes **3.**

Selon un mode de réalisation, le ou les dispositifs optiques **4** sont disposés à une distance de détection **H40** des zones de tests **31.** De la sorte la distance de lecture des segments d'analyse **32a, 32b, 32c** est déterminée et reproductible. La distance **H40** peut être déterminée en fonction des caractéristiques de détection des dispositifs optiques **4.** Elle peut être par exemple comprise entre 0,5 mm et 50 mm, de préférence de l'ordre de 10 mm à 40 mm. La distance de lecteur **H40** peut en outre être déterminée en fonction du nombre de bandelettes à lire et/ou de leurs dimensions et/ou de leur espacement dans le logement **12.**

Selon un mode de réalisation, les bandelettes **3** sont sont disposées ou prédisposées sur un test **2** (figure 1). Les bandelettes **3** peuvent alors être disposées sur le test **2** à des emplacements dédiés **22a, 22b, 22c, 22d, 22e.** Les emplacements dédiés peuvent par exemple prendre la forme d'une surface ou d'un évidement dans une surface du test **2** où les bandelettes **3** libres peuvent être disposées. Des moyens de maintien tels que des ergots peuvent être prévus pour maintenir les bandelettes sur le test **2** à leur emplacement dédié. Le test **2** comporte un cadre **21** dont les formes et les dimensions correspondent à celles du logement **12.** Le test **2,** peut ainsi être inséré dans le logement **12.** Les bandelettes **3** disposées sur le test **2** se trouvent alors disposées en vis-à-vis du ou des dispositifs optiques **4** de sorte à pouvoir être lues.

Selon un mode de réalisation, le test **2** peut être directement inséré dans une ouverture **120** pour disposer les bandelettes **3** dans le logement **12.** L'insertion peut être guidée par des glissières ou d'autres éléments de guidage. Un moyen de verrouillage peut être prévu pour maintenir le test 2 en position dans le logement **12.** Un tel moyen de verrouillage peut prendre la forme d'un ou plusieurs ergots ou de clips ou d'un système aimanté ou tout équivalent. Le cas échéant, un couvercle peut ensuite occulter l'ouverture **120** pour procéder à la mesure. Le couvercle peut en outre servir de moyen de verrouillage. Selon une autre disposition une paroi pivotable ou amovible du boitier peut être utilisée pour disposer le test **2,** laquelle est ensuite remise en place pour refermer le logement **12.** Dans le cas d'un mécanisme à tiroir, le test **2** peut être disposé sur la partie translatable du tiroir et être inséré dans le logement **12** en refermant le tiroir.

Selon un mode de réalisation, le test **2** est de forme globalement plane, de sorte que toutes les bandelettes **3** sont disposées dans un plan. Cela n'exclut cependant pas d'autres dispositions, per exemple cylindrique, où les bandelettes **3** sont disposées de façon circulaire et où les dispositifs optiques **4** sont disposés en périphérie en vis-à-vis des surfaces de lecture des bandelettes **3.**

Selon un mode de réalisation, le test **2** est inséré dans le boitier **10** sur une fraction de sa longueur. Une butée peut être prévue dans le boitier **10** pour limiter la course des tests **2** analysés, de sorte qu'une portion reste accessible à l'extérieur du boitier **10.** Une portion de l'ordre de 5% à 30% de la longueur des tests 2, typiquement 7%, 10%, 15% ou 20% de sa longueur, peut rester accessible à l'extérieur du boitier **10.** Ainsi, la manipulation du dispositif de mesure **1** reste ergonomique. Les tests **2** peuvent aisément être retirés du boitier après analyse, y compris avec des gants.

Selon un autre mode de réalisation, illustré par la figure **4****,** une bandelette **3** est disposée dans une capsule **34** comportant une fenêtre de lecture. La fenêtre de lecture est située au niveau de la zone de test 31 pour permettre la lecture des segments d'analyse. La capsule **34** peut alors disposée de sorte à faire coïncider la fenêtre de lecture avec un dispositif optique **4.** Une telle capsule **34** peut par exemple être disposée dans un évidement **12a, 12b** faisant office de logement. La forme tridimensionnelle de l'évidement peut être déterminée de sorte à être complémentaire de celle de la capsule **34.** Ainsi, la capsule **34** s'emboite dans le logement **12** prévu à cet effet. L'évidement **12a, 12b** peut comporter une ouverture en vis-à-vis d'un dispositif optique **4** disposé dans le boitier **10.** La capsule **34** est alors disposée dans le logement de sorte à placer la fenêtre de lecture face à l'ouverture de sorte que le dispositif optique **4** puisse lire la bandelette **3.** Selon ce mode de réalisation, la capsule **34** peut être considérée comme un support de test, à l'instar du test **2.**

Dans le cas où plusieurs évidements **12a, 12b,** sont prévus dans le boitier **10,** ils peuvent avoir des formes tridimensionnelles différentes, chacune adaptée pour accueillir une capsule **34** de forme tridimensionnelle spécifique. Une capsule **34** est utilisée pour la détection d'une substance spécifique. Alternativement, une capsule **34** peut être prévue pour détecter plus d'une substance. Plusieurs capsules **34** peuvent être disposées dans plusieurs évidements pour une lecture simultanée.

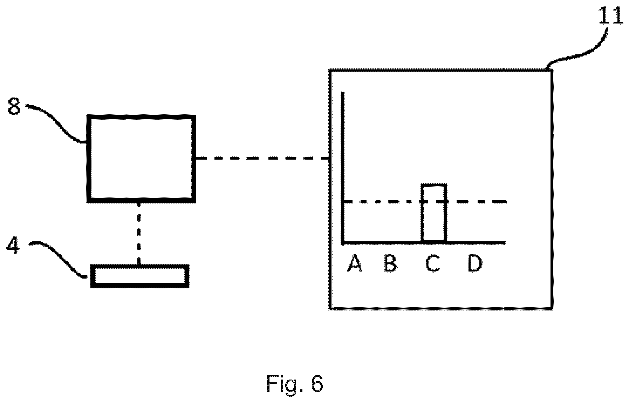

L'analyseur **1** selon la présente invention comporte une ou plusieurs unités **8** de traitement des signaux transmis par le ou les dispositifs optiques **4** (figure 6). L'unité de traitement **8** est programmée ou configurée pour identifier la couleur et ou l'intensité des segments d'analyse **32a, 32b, 32c.** L'unité de traitement peut en outre être adaptée à détecter la présence ou l'absence d'un test **2** dans le logement **12.** L'unité de traitement **8** peut en outre permettre l'identification automatique des bandelettes **3** en fonction de leurs segments d'analyse **32a, 32b, 32c** quelle que soit leur emplacement dans le logement **12.** Les bandelettes 3 peuvent ainsi être disposées dans le logement **12** de façon aléatoire. Alternativement, chaque bandelette **3** est disposée dans le logement **12** de faon prédéterminée, sa position étant déterminée par le type de substance pour laquelle il est dédié.

Selon un mode de réalisation, l'unité de traitement **8** permet de comparer les signaux d'analyse à des signaux de référence. Les signaux de référence peuvent être enregistrés dans une mémoire intégrée à l'analyseur **1.** Les signaux de référence comportent par exemple un ou plusieurs ensembles de segments d'analyse de référence, dont la couleur et/ou l'intensité témoigne de la présence d'une substance déterminée ou de sa concentration. Les signaux de référence peuvent comporter la séquence de segments d'analyse correspondant à la présence d'une substance donnée. Ils peuvent alternativement ou en plus comporter le séquence de segment d'analyse correspondant à l'absence d'une telle substance. Ils peuvent en outre ou alternativement comporter un ou plusieurs seuils de concentrations au-delà desquels la substance est considérée comme présente et effectivement absorbée par la personne testés. Des seuils élevés de concentration peuvent être déterminés pour alerter sur d'éventuels risques de santé. Les signaux de référence peuvent comporter les séquences de segments d'analyse de plusieurs types de bandelettes **3** pouvant être utilisées à cette fin. Les bandelettes **3** utilisables sont de préférence reconnues et qualifiées pour leur fiabilité et/ou leur validité réglementaire. Le type de bandelettes **3** utilisées peut ainsi être automatiquement reconnu par les signaux d'analyse. Alternativement, une sélection manuelle peut être effectuée avant l'analyse de sorte à préciser le type de bandelettes utilisées et/ou le type de substance recherchée.

Selon un mode de réalisation les tests **2** sont préconditionnés avec une ou plusieurs bandelettes **3.** Différents tests **2** comprenant des assortiments de bandelettes **3** spécifiques peuvent être acquis.

Les signaux de référence des substances illicites ou à rechercher sont stockés dans une mémoire ou une base de données de sorte à pouvoir être comparés aux signaux d'analyse d'un ou plusieurs échantillons **6.** Selon un mode de réalisation, chacun des signaux détectés est comparé à l'ensemble des signaux de référence de la base de données. Alternativement, les signaux d'analyse sont comparés à un jeu de signaux de référence correspondant à la substance recherchée et/ou au type de test utilisé.

L'unité ou les unités de traitement **8** peuventt mettre en œuvre les algorithmes adéquats permettant de déterminer le degré de similitude avec des signaux de référence. De préférence, les signaux d'analyse sont comparés avec des signaux de référence correspondant à l'absence de la substance recherchée et des signaux de référence correspondant à la présence de la substance recherchée ou à un seuil de concentration. En fonction du degré de correspondance avec l'un ou l'autre de ces signaux de référence, le test peut être jugé positif ou négatif. D'autres méthodes statistiques comportant par exemple la pondération des signaux d'analyse et/ou leurs moyennes peuvent être utilisées, notamment lorsque plusieurs dispositifs optiques **4** sont impliqués pour des mesures redondantes ou lorsque plusieurs longueurs d'onde sont émises par les sources lumineuses. L'unité de traitement **8** peut comporter ou être connectée à une intelligence artificielle permettant notamment l'apprentissage sur la base d'échantillons validés comme positifs ou négatif par d'autres moyens de mesures.

L'unité ou les unités de traitement **8** permet de déterminer avec certitude le résultat de l'analyse, de sorte qu'il ne soit pas soumis à l'interprétation de l'opérateur. Le résultat de l'analyse, émis par l'unité de traitement **8** peut être affiché en temps réel sur un écran **11** de l'analyseur **1.** L'affichage peut comporter la présence d'une ou plusieurs substances recherchées si la lecture des tests correspondants est positive. Il peut en outre ou alternativement comparer le résultat à un ou plusieurs seuils de concentrations. Il peut en outre ou alternativement comporter l'absence des substances pour lesquelles les tests se sont révélés négatifs. L'affichage peut en outre ou alternativement préciser la présence ou l'absence de test **2** ou de bandelettes **3** dans le logement **12** pour les substances recherchées. Par exemple, un ensemble de substances officiellement recherchées peuvent être stockées dans la mémoire de l'analyseur **1** ou dans une base de données, de sorte que les signaux d'analyse sont systématiquement comparés à l'ensemble des substances recherchées. Si le nombre de tests **3** présents dans le logement **12** ne permet pas d'analyser l'ensemble des substances recherchées, l'affichage peut mentionner les substances n'ayant pas fait l'objet d'une analyse. Similairement, si un test non reconnu ou non conforme est utilisé, l'affichage peut mentionner que les substances correspondantes ne sont pas considérées comme effectivement analysées.

Selon un mode de réalisation, l'analyseur **1** comporte un ou plusieurs organes de connexion **13.** Il peut s'agir de connexion électrique, notamment pour l'alimentation électrique du dispositif à partir d'une source extérieure. L'organe de connexion peut ainsi servir à recharger une batterie du dispositif de mesure ou à l'alimenter directement. L'organe de connexion peut alternativement ou en plus servir à transmettre des données d'analyse à d'autres appareils. Par exemple, une imprimante **7** (figure 5) peut être prévue pour imprimer le résultat d'analyse sur un support physique **71** tel qu'un papier. En l'occurrence, un procès verbal contenant les résultats d'analyse peut être imprimé et directement transmis à la personne testée et jugée contrevenante. Alternativement ou en plus, des moyens de communication à distance des données peuvent être prévus, basés par exemple sur des protocole wifi, Bluetooth, ou téléphoniques. La communication des données d'analyse peut cependant rester non transmissibles pour des raisons de confidentialité.

L'analyseur **1** peut comporter un dispositif de géolocalisation basé par exemple sur le GPS ou tout équivalent. L'analyse peut être corrélée à un lieu. L'heure de l'analyse peut en outre être enregistrée.

L'analyseur **1** peut comporter selon les besoins un pavé numérique ou des touches de sélection permettant de choisir différentes options d'un menu affiché sur l'écran **11,** un dispositif d'activation et d'inactivation du dispositif ou tout autre interface homme-machine utile. Des données relatives à l'identité d'une personne testée peuvent ainsi être implémentées et associées à une analyse.

L'analyseur **1** selon la présente invention est de dimension réduite facile à manipuler. La longueur est typiquement inférieure à 300 mm, voir 250 mm. De préférence la longueur est au maximum de 210mm. Elle est par exemple comprise entre 100 et 207 mm. La largeur est inférieure à 200 mm voir 150 mm. De préférence, la largeur est au maximum de 100 mm. Elle peut être comprise par exemple entre 50 et 100 mm. L'épaisseur est typiquement de l'ordre de 30 à 80 mm. De préférence l'épaisseur est au maximum de 55 mm. Selon le relief de l'analyseur seconde épaisseur peut être définie, étant au maximum de 40 mm. Le dispositif peut par exemple être manipulé d'une main. Son ergonomie est adaptée à l'usage sur le terrain par des utilisateurs non nécessairement formés pour pratiquer des tests biologiques. Le dispositif est en l'occurrence intuitif et automatisé. En particulier, un utilisateur peut l'utiliser correctement avec peu d'instruction. Typiquement un utilisateur ayant collecté un échantillon à analyser peut tout simplement l'insérer dans le boitier et lire le résultat de mesure.

Selon un mode de réalisation, le dispositif ici décrit peut être utilisé en laboratoire le cas échéant, seul ou en combinaison avec d'autres instruments de détection et de mesure.

Le positionnement des tests **3** dans le logement **12** est stable et reproductible. En l'occurrence, une mesure peut être effectuée quelle que soit l'orientation ou l'inclinaison du dispositif de mesure. En effet, les bandelettes **3** sont maintenues à une distance de détection **H40** reproductible du fait du positionnement du test **2.**

Selon un mode de réalisation, le ou les dispositifs optiques 4 sont en mesure de déterminer si une bandelette **3** ou un test **2** n'est pas dans une position adéquate, par exemple à une distance de détection **H40** requise. En cas de mauvais positionnement, une alerte peut être produite, visuelle et/ou sonore.

Selon un mode de réalisation, un emplacement peut être dédié dans le logement **12,** à un test de référence. Un tel test de référence peut par exemple permettre de confirmer le bon fonctionnement des organes de détection, en particulier du ou des dispositifs optiques **4** et de l'unité de traitement **8** au moment de l'analyse. Un tests de référence peut à cet effet comporter une ou plusieurs couleurs de référence ou d'autres éléments de référence. Un test de référence peut servir par exemple à confirmer que la calibration du dispositif de mesure est conforme aux exigences. Il peut alternativement servir à calibrer l'analyseur **1.**

La présente invention couvre en outre un système de mesure comprenant un analyseur **1** selon la présente invention. Un tel système comporte outre l'analyseur **1,** un ou plusieurs accessoires, en l'occurrence une imprimante miniaturisée **7** permettant d'imprimer le rapport d'analyse sur un support physique tel qu'un papier.

Le système peut comporter un kit de tests **2,** également objet de la présente invention. Un tel kit de tests **2** permet de détecter la présence de plusieurs substances recherchées de manière simultanée.. Les tests **2** peuvent être référencés de sorte que les substances qu'ils visent à identifier soient aisément connues de l'utilisateur. Les références nécessaires peuvent être inscrites sur l'emballage ou directement sur le test de manière explicite pour une lecture directe par l'utilisateur ou via un système d'identification numérique tels qu'un QR code ou une puce RFID. Le kit peut en outre comporter un ou plusieurs éléments de kit tel que récipient, réactifs liquides, bâtonnets, pipettes ou tout autre ustensile nécessaire à la mise en œuvre de l'analyse. Un échantillon de salive ou d'urine peut par exemple être recueilli dans un récipient pour être dispensé directement sur différents tests **2** ou plusieurs bandelettes **3** d'un test **2.** Alternativement, un bâtonnet de prélèvement peut être frotté sur les muqueuses et immergé dans un réactif liquide pour y solubiliser les éventuelles substances à rechercher. Le réactif liquide peut ensuite être dispensé sur les bandelettes des tests **2.** Le kit selon la présente invention peut en outre comprendre un mode d'emploi, soit sur papier soit enregistré dans une mémoire de sorte à pouvoir être affichée sur l'écran de l'analyseur **1.** De préférence, le mode d'emploi est traduit en différentes étapes qui s'affichent sur l'écran **11** de l'analyseur **1.** Chacune des étapes de l'analyse peut faire l'objet d'une confirmation de l'utilisateur de sorte que l'étape suivante s'affiche sur l'écran **11.** Ainsi, le procédé est respecté et effectué de manière reproductible et fiable.

Le système peut comporter un étui ou un contenant adapté à transporter l'analyseur **1,** ainsi que ses éventuels accessoires et éléments de kit. Un tel étui est de préférence rigide. Il peut comporter en outre des espaces prédéfinis pour y loger les différents éléments.

La présente invention couvre également un procédé d'analyse de plusieurs substance prédéfinies de façon simple et fiable sur le terrain. Le procédé peut s'effectuer au moyen de l'analyseur **1** décrit ci-dessus, avec l'aide d'éventuels éléments de kit de détection. Le procédé de détection comporte une étape de prélever un échantillon **6.** L'échantillon est de préférence un liquide corporel, en particulier de la salive ou de l'urine. L'échantillon 6 peut être analysé directement ou bien subir une ou plusieurs étapes de préparation supplémentaires. Il peut être par exemple mélangé à un réactif ou un solvent.

Le procédé comporte l'étape de collecter l'échantillon de liquide biologique sur au moins une bandelette d'un test, de préférence plusieurs bandelettes **3** telles que décrites plus haut. De préférence les tests **2** permettent de déterminer la présence et/ou la concentration d'un ensemble de substances prédéterminées.

Le procédé comporte une étape d'insérer le test **2** comprenant la ou les bandelettes imbibées de l'échantillon **6** dans l'analyseur, ou de sorte à être lus par l'analyseur **1.** Les bandelettes **3** sont disposées sur un test **2** au préalable.

Le procédé comporte l'étape de lire le test **3** au moyen d'un dispositif digital de sorte à s'affranchir d'éventuelles erreurs de lecture d'un opérateur humain. Le dispositif optique **4** et l'unité de traitement **8** mentionnés ci-dessus peuvent être utilisés à cette fin. Le dispositif digital est de préférence l'analyseur ici-décrit.

Le procédé comporte l'étape de traiter de manière digitale les signaux reçus et de produire un résultat de test tel que décrit plus haut.

Le procédé comporte l'étape d'afficher le résultat de test sur un écran **11** et/ou de le transmettre à un appareil accessoire **7.**

Selon un mode de réalisation, le procédé comporte une étape de calibration de l'analyseur **1.** La calibration peut être effectuée en amont de son utilisation pour la détection de substances, par exemple lors de son initialisation ou lors d'une mise à jour à distance. La calibration peut comporter l'étape d'enregistrer dans une mémoire ou une base de données des signaux de référence pour plusieurs substances recherchées. De telles substances sont de préférences celles listées de manière officielle en fonction des législations en vigueur. L'enregistrement de tels signaux de référence peut se faire via la lecture de tests **3** déterminés come étant positifs ou négatifs ou comme indiquant une concentration de référence. Ainsi, une série de plusieurs tests peut être enregistrée et référencée dans la mémoire de l'analyseur **1** ou dans une base de données. Alternativement, la calibration peut s'effectuer à distance lors de la connexion du dispositif de mesure à une base de données centrale où les signaux correspondant aux substances à rechercher sont régulièrement mis à jour.

### Numéros de référence employés sur les figures

- 1: Analyseur
- 10: Boitier
- 11: Ecran
- 12, 12a, 12b: Logement
- 120: Ouverture de logement
- 13: Organe de connexion électrique ou digitale
- 2: Test
- 21: Cadre de support
- 22a, 22b, 22c, 22d, 22e: Emplacements de tests
- 3: Bandelette
- 31: Zone de test
- 32a, 32b, 32c: Segments d'analyse
- 34: Capsule
- 4: Dispositif optique
- 5: Source lumineuse
- 6: Echantillon
- 7: Accessoire
- 71: Support d'impression
- 8: Unité de traitement
- H40: Distance de détection

## Revendications

1. Analyseur (1) de plusieurs substances prédéterminées comprenant :
- un boitier (10) au sein duquel un logement (12) est aménagé,
- un ou plusieurs dispositifs optiques (4) disposés dans le logement (12),
- au moins un test (2), comprenant une ou plusieurs bandelettes (3),
- une ou plusieurs sources lumineuses (5) adaptées à illuminer ledit au moins un test (2) disposés dans le logement (12), et
- une ou plusieurs unités de traitement (8) des signaux du ou des dispositifs optiques (4),
**caractérisé en ce que** le ou les dispositifs optiques (4) sont adaptés à lire plusieurs bandelettes (3) de manière simultanée et **en ce que** ledit test (2) est adapté à maintenir plusieurs bandelettes (3) à une distance de détection (H40) du ou des dispositifs optiques (4) quelle que soit l'orientation de l'analyseur (1).

2. Analyseur selon la revendication 1, ledit logement (12) comprenant une ouverture (120) dans le boitier (10) obturée par un couvercle.

3. Analyseur selon l'une des revendications 1 et 2, ledit test (2) comprenant un cadre (22), plusieurs emplacements (22a, 22b, 22c, 22d, 22e) adaptés pour accueillir chacun une bandelette (3) et au moins un moyen de maintien des bandelettes à leur emplacement.

4. Analyseur selon l'une des revendications 1 à 3, ledit test (2) étant adapté à disposer plusieurs bandelettes dans un plan.

5. Analyseur selon l'une des revendications 1 à 4, comprenant en outre une mémoire configurées avec des signaux de références, Lesdites une ou plusieurs unités de traitement comprenant des algorithmes de comparaison des signaux d'analyse avec lesdits signaux de référence.

6. Analyseur selon l'une des revendications 1 à 5, le ou les dispositifs optiques (4) étant configurés pour lire plus d'une bandelette (3), et/ou disposés de sorte que lesdites bandelettes (3) puissent être lues par plusieurs dispositifs optiques (4) de manière redondante.

7. Système comprenant un analyseur selon l'une des revendications 1 à 6, un ou plusieurs accessoires et un kit d'analyse comprenant au moins un élément parmi :
- un test (2) adapté à identifier la présence et/ou la concentration de plusieurs substances de manière simultanée,
- un ou plusieurs récipients apte à collecter un fluide corporel,
- un ou plusieurs réactifs ou solvants, et
- Un ou plusieurs ustensiles de manipulation

8. Système selon la revendication 7, lesdits accessoires comprenant une imprimante miniaturisée (7) avec un support d'impression physique (71).

9. Procédé d'analyse de plusieurs substances prédéterminées comprenant les étapes de :
- Collecter un échantillon (6) à partir d'un fluide corporel,
- Dispenser l'échantillon (6) sur une ou plusieurs bandelettes (3) d'un test (2), adaptées à l'identification desdites substances,
- Lire lesdites bandelettes de manière simultanée au moyen d'un analyseur selon l'une des revendications 1 à 7, et
- Produire le résultat de l'analyse, incluant la ou les substances détectées et la ou les substances non détectées.

10. procédé selon la revendication 9, comprenant en outre une étape de calibration, lors de laquelle plusieurs signaux de référence correspondant auxdites substances sont stockés dans une mémoire ou une base de données en vue d'être comparées aux signaux d'analyses.
